# EUROPEAN PATENT APPLICATION

(11) **EP 4 434 473 A1**
(43) Date of publication of application: **25.09.2024**
(21) Application number: 22895184.4
(22) Date of filing: 26.08.2022
(51) Int. Cl.: A61B 8/14

(54) **MEDICAL DEVICE**

(30) Priority: 19.11.2021 JP 2021188383
(71) Applicant: Asahi Intecc Co., Ltd., Seto-shi, Aichi 489-0071 (JP)
(72) Inventor: HIRANO Keisuke, Seto-shi, Aichi 489-0071 (JP); OSHIMA Fumiyoshi, Seto-shi, Aichi 489-0071 (JP)
(74) Representative: Prinz & Partner mbB
(86) International application number: PCT/JP2022/032174
(87) International publication number: WO 2023/089896

(57) **Abstract**

A medical apparatus includes an ultrasonic information acquisition portion that acquires ultrasonic information, inside a living body, which is obtained from an ultrasonic probe that includes an ultrasonic sensor and applies ultrasonic waves into the living body, an echo image generation portion that generates an ultrasonic echo image representing a longitudinal section of a blood vessel from the ultrasonic information, a guide image generation portion that generates a guide image including a guideline extending along a center line of the blood vessel appearing in the ultrasonic echo image from the ultrasonic information, and a display control portion that generates and displays a composite image in which the ultrasonic echo image and the guide image are superimposed.

## Description

### TECHNICAL FIELD

The present invention relates to a medical apparatus.

### BACKGROUND ART

A known technology acquires information on the inside of the human body from the human body using ultrasonic waves. For example, Patent literature 1 discloses an ultrasonic diagnostic apparatus that generates functional volume data, cross-sectional images, and MPR images of a subject based on position and angle information and echo data (ultrasonic information) from an ultrasonic probe. For example, Patent literature 2 discloses an ultrasonic observation apparatus that uses ultrasonic information based on the operation of an ultrasonic probe to display guide information that guides a treatment using a treatment instrument.

### CITATION LIST

### Patent Literature

Patent Literature 1: JP 2018-50655 A
Patent Literature 2: JP 2017-176465 A

### SUMMARY OF INVENTION

### TECHNICAL PROBLEM

For example, a known method in an intervention for the lower limb performs a procedure while observing a blood vessel and a medical device such as a guide wire or a catheter in the blood vessel in real time using ultrasonic echo images generated from ultrasonic information (hereinafter also referred to as an "echo-guided intervention"). In such an interventional procedure, the medical device is preferably delivered to the target position without touching the vascular wall in order to prevent damage to the vascular wall. However, in the echo-guided intervention, it is necessary to deliver the medical device while confirming the position and shape of the blood vessel, the position and orientation of the medical device, and the like appearing in the ultrasonic echo images. This causes a problem of requiring a high level of skill.

Further, if there is a tissue with a relatively high reflectance of ultrasonic waves (e.g., a bone, a calcified lesion, etc.) in the travel direction of the ultrasonic waves emitted from the ultrasonic probe, the ultrasonic waves may be totally reflected by such a tissue before reaching the blood vessel, causing an issue such as the blood vessel appearing in the ultrasonic echo image becoming blurred, or a part of the blood vessel not appearing in the ultrasonic echo image. In such a case, it becomes more difficult to deliver the medical device to the target position without the medical device touching the vascular wall even in the echo-guided intervention.

In this regard, the technologies described in Patent literatures 1 and 2 only display the insertion route (treatment instrument route) of the puncture needle from the body surface without giving any consideration to the delivery of the guidewire, the catheter, or the like in the echo-guided intervention. Note that these issues are not limited to interventions for lower limbs, but also common to echo-guided interventions for other blood vessels such as the coronary arteries, the cerebral arteries, and the carotid arteries. Further, these issues are not limited to the vascular system, but also common when inserting and delivering the medical device, under echo guidance, to various organs in the human body, such as the lymphatic system, the biliary tract system, the urinary tract system, the respiratory tract system, the digestive system, the secretory glands, and the reproductive organs. In addition, it is required to improve the usability of the medical apparatus.

The present invention has been made to solve at least a part of the above-mentioned problems, and an object of the present invention is to provide a technology that can guide a medical device through a delivery route in an echo-guided intervention.

### SOLUTION TO PROBLEM

The present invention has been made to solve at least a part of the above-mentioned problems and can be implemented as the following aspects.
(1) According to one aspect of the present invention, a medical apparatus is provided. This medical apparatus includes: an ultrasonic information acquisition portion that acquires ultrasonic information in a living body obtained from an ultrasonic probe that includes an ultrasonic sensor and applies ultrasonic waves into the living body; an echo image generation portion that generates an ultrasonic echo image representing a longitudinal section of a blood vessel from the ultrasonic information; a guide image generation portion that generates a guide image including a guideline extending along a center line of the blood vessel appearing in the ultrasonic echo image from the ultrasonic information; and a display control portion that generates and displays a composite image in which the ultrasonic echo image and the guide image are superimposed.
   According to this configuration, the display control portion displays the composite image in which the ultrasonic echo image and the guide image including the guideline are superimposed, making it possible to guide the medical device through the delivery route in the echo-guided intervention. Further, the guideline extends along the center line of the blood vessel appearing in the ultrasonic echo image. This allows an operator to deliver the medical device to the target position without the medical device touching the vascular wall simply by delivering the medical device along the guideline. Thus, the safety and efficiency of the procedure can be improved. Further, the guideline is drawn by the guide image generation portion. Thus, the guideline is not interrupted in case an issue occurs such as the blood vessel appearing in the ultrasonic echo image becomes blurred, or a part of the blood vessel not appears in the ultrasonic echo image, due to, for example, the presence of tissues with a relatively high reflectance of ultrasonic waves (bones, calcified lesions, etc.) in the travel direction of the ultrasonic waves emitted from the ultrasonic probe. Thus, according to the present configuration, the safety and efficiency of the procedure can be improved without relying on information in the ultrasonic echo image.
(2) In the medical apparatus of the above aspect, the guide image generation portion may change the width of the guideline according to the inner diameter of the blood vessel.
   According to this configuration, the guide image generation portion changes the width of the guideline according to the inner diameter of the blood vessel, making it possible to reduce the width of the guideline in a part where the inner diameter of the blood vessel is relatively small, and increase the width of the guideline in a part where the inner diameter of the blood vessel is relatively large. This allows the operator to quickly recognize, based on the width of the guideline, a part where the medical device needs to be advanced carefully (i.e., a part where the inner diameter of the blood vessel is relatively small) and a part where the medical device can be advanced normally (i.e., a part where the inner diameter of the blood vessel is relatively large). As a result, according to the present configuration, the safety and efficiency of the procedure can be further improved.
(3) In the medical apparatus of the above aspect, the guide image generation portion may set the width of the guideline within a range of 40% or more and 60% or less of the inner diameter of a corresponding part of the blood vessel.
   According to this configuration, the guide image generation portion sets the width of the guideline within the range of 40% or more and 60% or less of the inner diameter of the corresponding part of the blood vessel. This allows the operator to recognize the guideline at a glance and deliver the medical device along the guideline relatively easily. This can also reduce the possibility of the medical device touching the inner wall of the blood vessel. Further, the width of the guideline is made narrower in the part where the inner diameter of the blood vessel is relatively small, and the width of the guideline is made thicker in the part where the inner diameter of the blood vessel is relatively large. This allows the operator to quickly recognize, based on the width of the guideline, the part where the medical device needs to be advanced carefully and the part where the medical device may be advanced normally, making it possible to further improve the safety and efficiency of the procedure.
(4) In the medical apparatus of the above aspect, the guide image generation portion may change the width of the guideline according to an outer diameter of the medical device to be inserted into the blood vessel.
   According to this configuration, the guide image generation portion changes the width of the guideline according to the outer diameter of the medical device. Thus, if the outer diameter of the medical device is relatively narrow, the width of the guideline can be made narrower, and if the outer diameter of the medical device is relatively thick, the width of the guideline can be made thicker. Thus, it is easy for the operator to visually recognize both the medical device and the guideline on the composite image. As a result, according to the present configuration, the safety and efficiency of the procedure can be further improved.
(5) In the medical apparatus of the above aspect, the guide image generation portion may make the width of the guideline thicker than the outer diameter of the medical device.
   According to this configuration, the guide image generation portion makes the width of the guideline thicker than the outer diameter of the medical device. This allows the operator to deliver the medical device to the target position without the medical device touching the vascular wall simply by delivering the medical device such that the medical device does not protrude from the guideline on the composite image, making it possible to further improve the safety and efficiency of the procedure.
(6) The medical apparatus of the above aspect may further include an operation acquisition portion that acquires an enlargement instruction operation or a reduction instruction operation of the width of the guideline from a user of the medical apparatus. The guide image generation portion may regenerate the guide image with the width of the guideline thickened in response to the enlargement instruction operation and regenerate the guide image with the width of the guideline narrowed in response to the reduction instruction operation. The display control portion may generate and display the composite image in which the ultrasonic echo image and the regenerated guide image are superimposed.
   According to this configuration, the width of the guideline can be changed according to the enlargement instruction operation or the reduction instruction operation from the operator (the user of the medical apparatus). Thus, the usability of the medical apparatus can be improved.
(7) In the medical apparatus of the above aspect, the guide image generation portion may generate the guide image including the guideline in which an outline is drawn as a line segment of a predetermined color and the inside of the outline is made transparent or translucent.

According to this configuration, the guide image generation portion draws the guideline in which the outline is drawn as the line segment of the predetermined color and the inside of the outline is made transparent or translucent, making it possible to prevent the guideline from interfering with recognition of the medical device on the composite image.

Note that the present invention can be implemented in various aspects. For example, the present invention can be implemented as an image display apparatus (medical apparatus) that generates and displays images, an image generation apparatus that generates images and sends the images to other devices, an inspection apparatus with a built-in function of an image display apparatus or an image generation apparatus, a medical system that includes these apparatuses, production methods of these apparatuses and system, computer programs that achieve functions of these apparatuses and system, and the like.

### BRIEF DESCRIPTION OF DRAWINGS

Fig. 1 is an explanatory diagram illustrating a configuration of a medical system of a first embodiment.
Fig. 2 is an explanatory diagram of an ultrasonic probe.
Fig. 3 is an explanatory diagram illustrating a configuration of the ultrasonic probe.
Fig. 4 is a flowchart showing an example of a processing procedure of guide processing.
Fig. 5 is a diagram showing an example of a guide screen.
Figs. 6A and 6B are diagrams showing an example of an ultrasonic echo image and a guide image.
Fig. 7 is a diagram describing processing for a missing part.
Fig. 8 is a diagram showing an example of a composite image display screen.
Figs. 9A and 9B are diagrams describing an operation of a guideline.
Figs. 10A and 10B are diagrams describing how to change a display mode.
Figs. 11A and 11B are diagrams showing an example of the ultrasonic echo image and the guide image.
Fig. 12 is a diagram showing an example of the composite image display screen.
Fig. 13 is an explanatory diagram illustrating a configuration of the medical system of a second embodiment.
Figs. 14A and 14B are diagrams showing an example of the guide image and the composite image display screen.
Fig. 15 is an explanatory diagram illustrating a configuration of the medical system of a third embodiment.
Fig. 16 is a flowchart showing an example of a processing procedure of the guide processing of the third embodiment.
Fig. 17 is a diagram describing specification of a device outer diameter.

### EMBODIMENTS OF THE INVENTION

### <First embodiment>

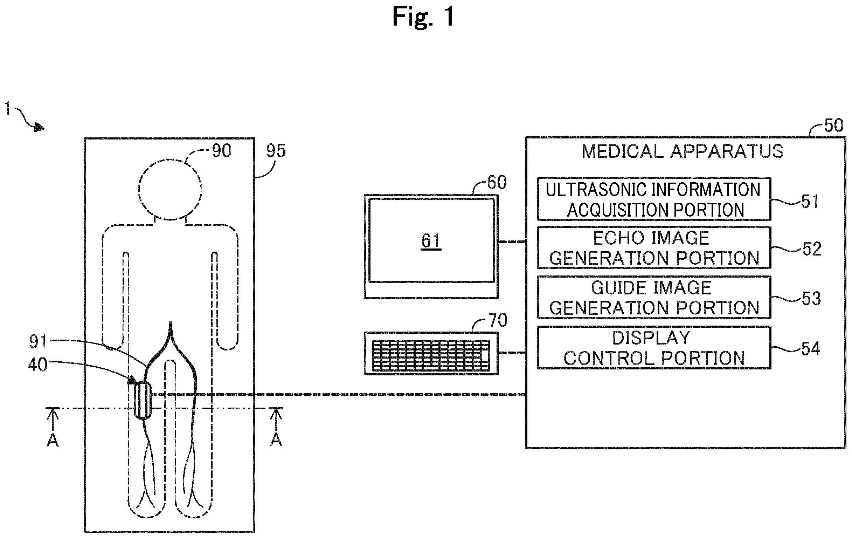

Fig. 1 is an explanatory diagram illustrating a configuration of a medical system 1 of a first embodiment. The medical system 1 is an apparatus used to treat or examine a living body (human body in this case) 90 to be treated, and includes an ultrasonic probe 40, a medical apparatus 50, a display portion 60, and an operation portion 70. The medical system 1 superimposes an ultrasonic echo image generated from ultrasonic information obtained from the ultrasonic probe 40 and a guide image showing a delivery route of the medical device, and displays the superimposed image. The medical system 1 can be used, for example, in an intervention for the lower limb, as a technique in which a procedure is performed using the ultrasonic echo image generated from the ultrasonic information while observing a blood vessel and a medical device such as a guide wire or a catheter inside the blood vessel in real time (hereinafter also referred to as an "echo-guided intervention"). Hereinafter, the blood vessel is described as an example of a biological duct. However, in addition to the vascular system, the medical system 1 is also applicable to the lymph gland system, the biliary tract system, the urinary tract system, the respiratory tract system, the digestive system, the secretory glands, the reproductive organs, and the like. Further, although a guide wire is described as an example of the medical device, any medical device such as a catheter can be used in addition to the guide wire.

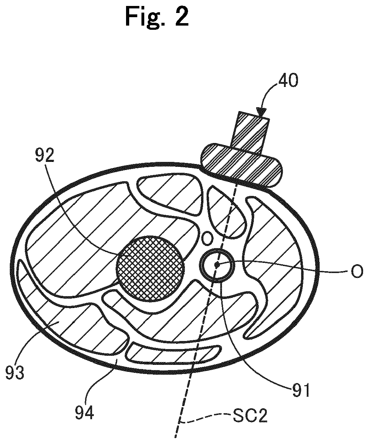

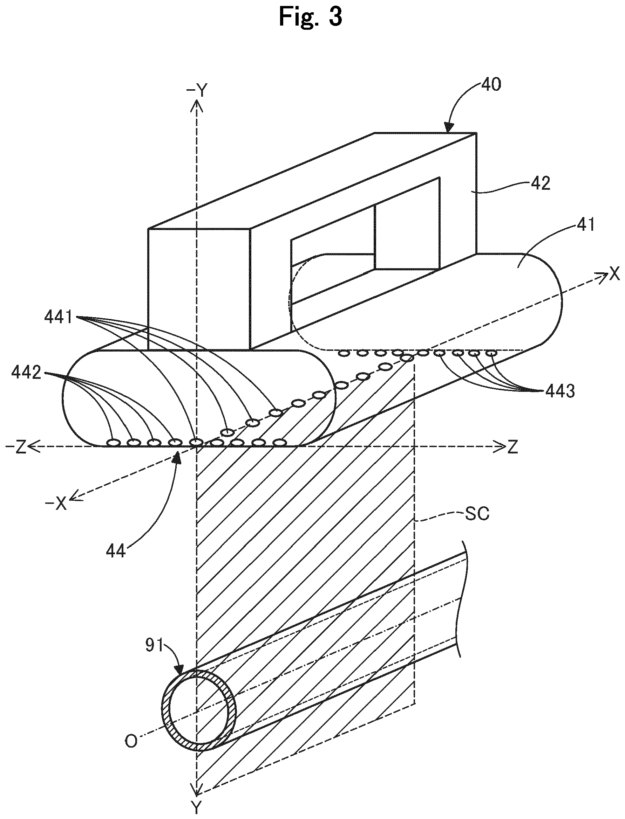

Fig. 2 is an explanatory diagram of the ultrasonic probe 40. Fig. 3 is an explanatory diagram illustrating a configuration of the ultrasonic probe 40. Fig. 2 shows a cross section taken along a line A-A in Fig. 1. Fig. 2 shows the ultrasonic probe 40 pressed against the thigh of the human body 90 (Fig. 1) lying on a bed 95, as well as a bone 92, a muscle 93, and a fat 94 surrounding a blood vessel 91. Fig. 3 shows mutually perpendicular X, Y, and Z axes. The X axis corresponds to a longitudinal direction of the ultrasonic probe 40 and an extension direction of the blood vessel 91. The Y axis corresponds to a height direction of the ultrasonic probe 40 and a height direction of the blood vessel 91. The Z axis corresponds to a width direction (transverse direction) of the ultrasonic probe 40 and a width direction of the blood vessel 91. The blood vessel 91 is a blood vessel in the thigh of the human body 90 (Fig. 1).

The ultrasonic probe 40 is a device that intermittently applies ultra-short ultrasonic pulses to the inside of the human body 90 including the blood vessels 91 and detects the time it takes to obtain a reflected wave (echo) and the intensity of the reflected wave (echo). The ultrasonic probe 40 includes a main body portion 41, a handle portion 42, and a plurality of ultrasonic sensors 44.

As shown in Fig. 3, the main body portion 41 is a casing that is brought into contact with a body surface (skin) of the human body 90. The handle portion 42 is a grip portion disposed on the surface of the main body portion 41 opposite to the side on which the ultrasonic sensors 44 are disposed, and is used by the operator to hold the ultrasonic probe 40. Note that the handle portion 42 may be held by a robot arm or the like. Further, the handle portion 42 may be omitted, and the robot arm may directly hold the main body portion 41. The main body portion 41 and the handle portion 42 can be formed using a resin material such as polyamide, polypropylene, polycarbonate, polyacetal, or polyether sulfone.

The ultrasonic sensor 44 is an ultrasonic probe (also referred to as an ultrasonic oscillator, a piezoelectric body, an ultrasonic transmitting/receiving element, or an ultrasonic element) that transmits ultrasonic waves toward a living tissue inside the human body 90, such as the blood vessels 91, and receives the reflected ultrasonic waves that have propagated through the living tissue. In the example of Fig. 3, the ultrasonic sensors 44 are arranged on the surface of the main body portion 41 opposite to the side where the handle portion 42 is disposed. Specifically, the ultrasonic sensors 44 include element groups 441, 442, and 443. The element group 441 is a plurality of elements arranged linearly along the longitudinal direction (X-axis direction) of the main body portion 41. The element group 442 is a plurality of elements arranged linearly along the lateral direction (Z-axis direction) of the main body portion 41 at one end (end in the -X-axis direction) of the element group 441. The element group 443 is a plurality of elements arranged linearly along the lateral direction (Z-axis direction) of the main body portion 41 at the other end (end in the +X-axis direction) of the element group 441.

In Fig. 3, a scanning plane of the element group 441 (i.e., a cross section of the ultrasonic echo image to be obtained) is represented as SC. In the guide processing described below, the operator places the ultrasonic probe 40 so that the scanning plane SC of the element group 441 of the ultrasonic probe 40 passes through a central axis O of the blood vessel 91. In other words, the scanning plane SC passes through the center of the lateral axis (Z axis) of the blood vessel 91. The element groups 442 and 443 are used for this positioning (positioning of the scanning plane SC and the central axis O). A known method can be used to perform the positioning of the ultrasonic probe 40 and the blood vessel 91 using the element groups 442 and 443.

Note that, in the example of Fig. 3, the element groups 441, 442, and 443 disposed in the main body portion 41 are each arranged in one row, but they may be arranged in multiple rows. For example, the element group 441 extending in the X-axis direction may be arranged in two or more rows in the Z-axis direction. Further, at least one of the element groups 442 and 443 may be omitted. Further, the outside of the ultrasonic sensors 44 (i.e., the surface of the main body portion 41 on the +Y-axis direction side) may be covered with an acoustic matching layer or an acoustic lens. The acoustic matching layer is a layer that adjusts the difference in acoustic impedance between the ultrasonic sensors 44 and the human body 90. The acoustic lens is a layer that focuses the ultrasonic waves in a slice direction to improve resolution.

Returning to Fig. 1, the description will be continued. The display portion 60 is a liquid crystal display including a display screen 61. The display portion 60 displays various screens in the guide processing described below. Note that the display portion 60 may be configured by a display device other than the liquid crystal display (e.g., a touch pad, a smart glass, a projector, etc.). The operation portion 70 is a keyboard and a mouse for inputting information to the medical apparatus 50. Note that the operation portion 70 may be configured by an input device other than the keyboard and the mouse (e.g., a microphone for acquiring voice input, a touch panel, a foot switch, etc.).

The medical apparatus 50 is an apparatus that controls the entire medical system 1, and is electrically connected to each of the ultrasonic probe 40, the display portion 60, and the operation portion 70. The medical apparatus 50 is configured by including a CPU, a ROM, and a RAM, not shown, and the CPU executes a computer program stored in the ROM, thereby controlling the entire medical apparatus 50, as well as each of the ultrasonic probe 40, the display portion 60, and the operation portion 70, connected to the medical apparatus 50. Further, the medical apparatus 50 achieves each function of an ultrasonic information acquisition portion 51, an echo image generation portion 52, a guide image generation portion 53, and a display control portion 54. Note that the medical apparatus 50 may be connected to other apparatuses (a server, etc.) via a network.

The ultrasonic information acquisition portion 51 acquires the ultrasonic information from the ultrasonic probe 40. The term "ultrasonic information" described herein refers to information (electrical signals) obtained by applying the ultrasonic waves into the human body 90 and information indicating the time and intensity of the reflected wave (echo) detected by the ultrasonic probe 40. The ultrasonic information is used to generate an ultrasonic echo image and a guide image in the guide processing described below. Details will be described below.

The echo image generation portion 52 uses the ultrasonic information to generate an ultrasonic echo image representing a longitudinal section of the blood vessel 91 (Fig. 3) using a known method in the guide processing described below. The guide image generation portion 53 specifies the position of the center line of the blood vessel 91 from the ultrasonic information and generates a guide image including a guideline extending along the specified center line in the guide processing described below. The display control portion 54 controls the entire guide process described below. Further, the display control portion 54 generates a composite image in which the ultrasonic echo image generated by the echo image generation portion 52 and the guide image generated by the guide image generation portion 53 are superimposed and causes the display portion 60 to display the composite image in the guide processing described below.

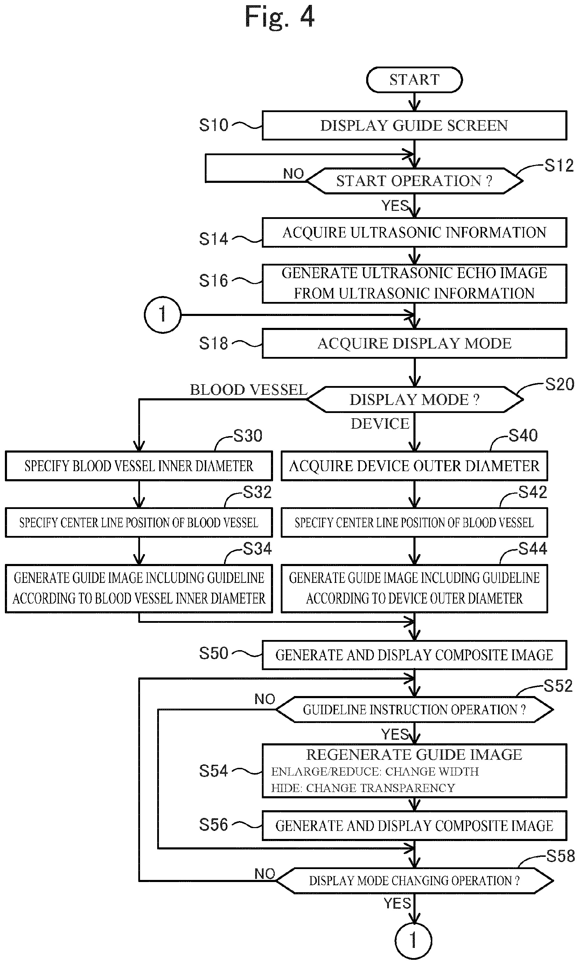

Fig. 4 is a flowchart showing an example of a processing procedure of guide processing. The guide processing is processing that generates and displays the composite image to guide a medical device through a delivery route. The guide processing can be started at any timing. For example, the guide processing can be initiated by activation of the medical apparatus 50.

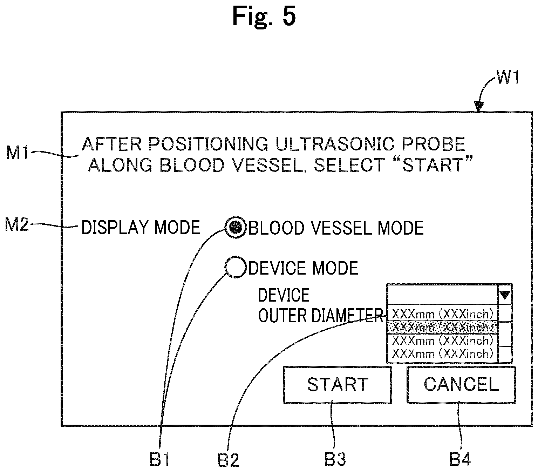

Fig. 5 is a diagram showing an example of a guide screen W1. In step S10 of Fig. 4, the display control portion 54 causes the display portion 60 to display the guide screen W1 (Fig. 5). The guide screen W1 includes a message M1 for prompting the placement of the ultrasonic probe 40, a message M2 for prompting the selection of the display mode, a display mode selection section B1, a device outer diameter selection section B2, a start button B3, and a cancel button B4. In the illustrated example, the selection section B1 is in a radio button format, allowing selection of either a blood vessel mode or a device mode. The term "blood vessel mode" described herein refers to a mode in which the width of the guideline is changed according to the inner diameter of the blood vessel 91. The term "device mode" refers to a mode in which the width of the guideline is changed according to the outer diameter of the medical device. In the illustrated example, the selection section B2 is in a list format, and the diameter of the medical device is listed in both millimeters and inches. Note that "X" in the selection section B2 means any given number.

The operator places the ultrasonic probe 40 along the blood vessel 91 according to the guide screen W1 displayed on the display portion 60 and aligns the ultrasonic probe 40 so that the scanning plane SC of the element group 441 passes through the central axis O of the blood vessel 91 (Fig. 3). Then, the operator operates the selection section B1 to select the desired display mode. When the device mode is selected, the operator selects the outer diameter of the medical device to be inserted into the blood vessel 91 by operating the selection section B2. The operator presses the start button B3. Note that if the cancel button B4 is pressed, the display control portion 54 closes the guide screen W1 and ends the processing.

In step S12 of Fig. 4, the display control portion 54 determines whether a start operation has been performed, that is, whether the start button B3 has been pressed. If the start operation has not been performed (step S12: NO), the display control portion 54 shifts the processing to the step S12 and waits for the start operation. If the start operation has been performed (step S12: YES), the display control portion 54 shifts the processing to a step S14. In the step S14, the ultrasonic information acquisition portion 51 acquires the ultrasonic information from the ultrasonic probe 40. The step S14 corresponds to an "acquisition step".

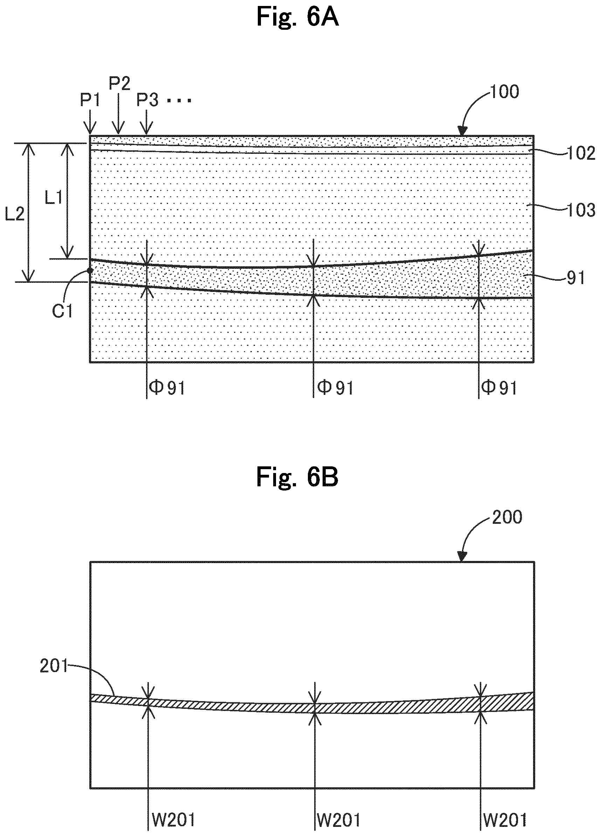

Figs. 6A and 6B are diagrams showing an example of an ultrasonic echo image 100 and a guide image 200. Fig. 6A shows an example of the ultrasonic echo image 100, and Fig. 6B shows an example of the guide image 200. In step S16 of Fig. 4, the echo image generation portion 52 generates a two-dimensional image, as the ultrasonic echo image 100, with gradations of light and shade depending on the intensity of the reflected wave of each element group 441 from the ultrasonic information acquired by the ultrasonic information acquisition portion 51. In the example of Fig. 6A, the ultrasonic echo image 100 includes a longitudinal section image of the blood vessels 91, a skin 102, and subcutaneous tissues 103 (tissues including a bone 92, a muscle 93, a fat 94, etc. in Fig. 2) on the scanning plane SC of the ultrasonic probe 40. Further, in the illustrated example, an inner diameter φ91 of the blood vessel 91 gradually increases from the left side to the right side. The step S16 corresponds to an "echo image generation step".

In step S18 of Fig. 4, the guide image generation portion 53 acquires the display mode (the blood vessel mode or the device mode) selected on the guide screen W1. In step S20, the guide image generation portion 53 shifts the processing to step S30 if the display mode is the blood vessel mode (step S20: blood vessel), and shifts the processing to step S40 if the display mode is the device mode (step S20: device).

The processing will be described for the case where the display mode is the blood vessel mode. In step S30 of Fig. 4, the guide image generation portion 53 specifies the inner diameter φ91 of the blood vessel 91. Specifically, the guide image generation portion 53 determine distances L1 and L2 at a given point P1 based on the ultrasonic information acquired by the ultrasonic information acquisition portion 51.
(a1) Distance L1: distance from the surface of the skin 102 to a vascular wall of the blood vessel 91 on the proximal side.
(a2) Distance L2: distance from the surface of the skin 102 to the vascular wall of the blood vessel 91 on the distal side.

Then, the guide image generation portion 53 sets a value obtained by subtracting the distance L1 from the distance L2 as the inner diameter φ91 of the blood vessel 91 at the point P1. The guide image generation portion 53 performs the similar processing while changing the calculation points P2 to Pn (n is an arbitrary natural number) at a predetermined interval, thereby specifying the inner diameter φ91 of the blood vessel 91 at each point. In this manner, the guide image generation portion 53 can acquire the inner diameter φ91 of the blood vessel 91 and the change in the inner diameter φ91.

In step S32 of Fig. 4, the guide image generation portion 53 specifies the position of the center line of the blood vessel 91. Specifically, the guide image generation portion 53 specifies a center point C1 between the vascular wall of the blood vessel 91 on the proximal side and the vascular wall on the distal side at the given point P1 based on the ultrasonic information acquired by the ultrasonic information acquisition portion 51. The guide image generation portion 53 performs the similar processing while changing the calculation points P2 to Pn at the predetermined interval, thereby specifying the center point C1 at each point. The guide image generation portion 53 specifies the position of a line segment connecting the center point C1 of each point thus specified as the position of the center line of the blood vessel 91. In this manner, in step S32, the position of the center line of the blood vessel 91 is specified using the ultrasonic information for generating the ultrasonic echo image 100. Thus, the position of the center line of the blood vessel 91 specified in step S32 is the position of the center line of the blood vessel 91 that appears in the ultrasonic echo image 100. Similarly, the inner diameter φ91 of the blood vessel 91 specified in step S30 is the inner diameter of the blood vessel 91 that appears in the ultrasonic echo image 100. Note that, as described in Fig. 3, when the scanning plane SC of the ultrasonic probe 40 is placed so as to pass through the central axis O of the blood vessel 91, the position of the center line of the blood vessel 91 specified in step S32 is the same as the position of the central axis O of the blood vessel 91.

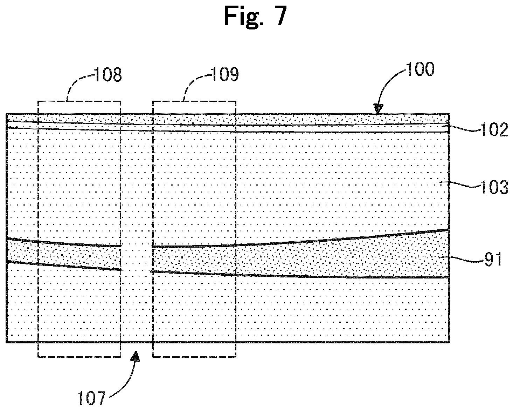

Fig. 7 is a diagram describing the processing if there is a missing part 107. If there is a tissue with a relatively high reflectance of ultrasonic waves (e.g., the bone 92, the calcified lesion, etc.) in the travel direction of the ultrasonic waves emitted from the ultrasonic probe 40, the ultrasonic waves may be totally reflected by such a tissue before reaching the blood vessels 91, causing a part (the missing part 107) without information in the ultrasonic information. As shown in Fig. 7, the ultrasonic echo image 100 generated using the ultrasonic information with the missing part includes a region where a part of the blood vessel 91 is not visible, and the vascular wall is hardly distinguished from other tissues. Further, the ultrasonic echo image 100 generated using the ultrasonic information with the missing part may include a region where the outline of the blood vessel 91 is blurred. In such a case, in step S32 of Fig. 4, the guide image generation portion 53 may interpolate the missing part 107 using information on surroundings 108 and 109 of the missing part 107 included in the ultrasonic information to specify the position of the center line of the blood vessel 91. A known technique can be used for this interpolation.

In step S34 of Fig. 4, the guide image generation portion 53 generates the guide image 200 including a guideline 201 according to the inner diameter φ91 of the blood vessel 91. Specifically, the guide image generation portion 53 draws the guideline 201 in which a width W201 is changed according to the change in the inner diameter φ91 of the blood vessel 91 specified in step S30, and generates an image, as the guide image 200, in which this guideline 201 is placed at the position of the center line of the blood vessel 91 specified in step S32. In this process, it is preferable that the guide image generation portion 53 sets the width W201 of the guideline 201 within a range of 40% or more and 60% or less of the inner diameter φ91 of a corresponding part of the blood vessel 91. If the width W201 of the guideline 201 is made smaller than 40% of the inner diameter φ91, there is a risk that the operator may not be able to recognize the guideline 201 at a glance. Further, the width W201 of the guideline 201 is narrow, making it difficult to deliver the medical device along the guideline 201 and causing a risk of reducing the efficiency of the procedure. On the other hand, if the width W201 of the guideline 201 is made larger than 60% of the inner diameter φ91, the operator can recognize the guideline 201 at a glance. However, the difference between the width W201 of the guideline 201 and the inner diameter φ91 of the blood vessel 91 is small, causing a risk that the medical device accidentally touches and damages the inner wall of the blood vessel 91. The width W201 of the guideline 201 is set within the range of 40% or more and 60% or less of the inner diameter φ91 of the corresponding part of the blood vessel 91, allowing the operator to visually recognize the guideline 201 at a glance and deliver the medical device along the guideline 201 relatively easy, and making it possible to reduce the possibility of the medical device touching the inner wall of the blood vessel 91. Further, the guide image generation portion 53 more preferably sets the width W201 of the guideline 201 to approximately 50% of the inner diameter φ91 of the corresponding part of the blood vessel 91. This makes it possible to achieve, in a well-balanced manner, facilitating the visibility of the guideline 201, facilitating the delivery of the medical device, and reducing the possibility of the medical device touching the inner wall of the blood vessel 91.

In the illustrated example, the guideline 201 is configured such that an outline is drawn with a line segment of a predetermined color, and the inside of the outline is filled with the same color. The predetermined color can be freely determined. However, since the ultrasonic echo image 100 has an achromatic color made of white and black, the predetermined color is preferably a chromatic color that can be distinguished from the ultrasonic echo image 100 at a glance. For example, the guideline 201 preferably has a light-yellow color that has higher visibility than the ultrasonic echo image 100. Note that the display format of the guideline 201 (e.g., hue, saturation, brightness, transparency, etc.) can be freely determined. Further, the inside of the outline of the guideline 201 may be filled in or may have a dot hatch or grid hatch pattern. Fig. 6B shows an example of the guide image 200 generated in the step S34. In the example of Fig. 6B, the guideline 201 has the width W201 that gradually increases from the left side to the right side, similar to the inner diameter φ91 of the blood vessel 91. The step S34 and a step S44 described below correspond to a "guide image generation step".

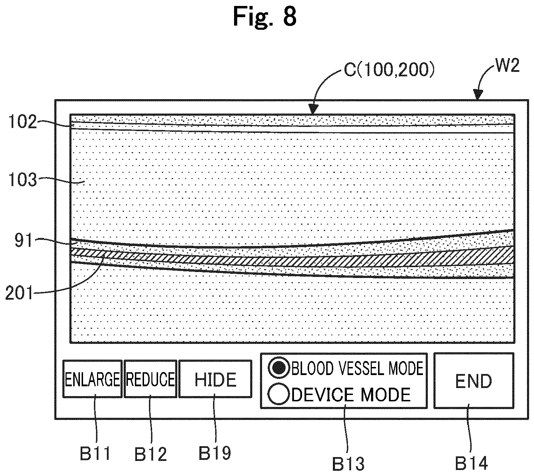

Fig. 8 is a diagram showing an example of a composite image display screen W2. In step S50 of Fig. 4, the display control portion 54 generates a composite image C in which the ultrasonic echo image 100 generated by the echo image generation portion 52 and the guide image 200 generated by the guide image generation portion 53 are superimposed, and causes the display portion 60 to display the composite image C. Specifically, the display control portion 54 generates the composite image display screen W2 including the composite image C and causes the display portion 60 to display the composite image display screen W2.

As shown in Fig. 8, the composite image display screen W2 includes the composite image C, an enlargement button B11, a reduction button B12, a hide button B19, a display mode selection section B13, and an end button B14. In the composite image C, the ultrasonic echo image 100 described in Figs. 6A and 6B and the guide image 200 are superimposed and displayed. In Fig. 8, for convenience of illustration, the composite image C is shown without the medical device. However, for example, when the operator inserts a medical device into the blood vessel 91, and the medical device is positioned on the scanning plane SC of the ultrasonic probe 40, the composite image C also includes an image of the medical device. The enlargement button B11 is a button used by the operator to perform an enlargement instruction operation of the width of the guideline 201. The reduction button B12 is a button used by the operator to perform a reduction instruction operation of the width of the guideline 201. The hide button B19 is a button used by the operator to perform a switching instruction operation between display/hide of the guideline 201. The selection section B13 is a radio button used by the operator to switch the display mode. In Fig. 8, a "blood vessel mode" is selected as the current display mode. The end button B14 is a button used by the operator to perform an end instruction operation of the processing. Note that the enlargement button B11, the reduction button B12, and the hide button B19 on the composite image display screen W2 function as an "operation acquisition portion". The step S50 corresponds to a "display control step".

In step S52 of Fig. 4, the display control portion 54 determines whether there has been an instruction operation for the guideline, that is, whether any of the enlargement button B11, the reduction button B12, and the hide button B19 has been pressed. If there is no instruction operation for the guideline (step S52: NO), the display control portion 54 shifts the processing to a step S58. If there is an instruction operation for the guideline (step S52: YES), the display control portion 54 shifts the processing to a step S54.

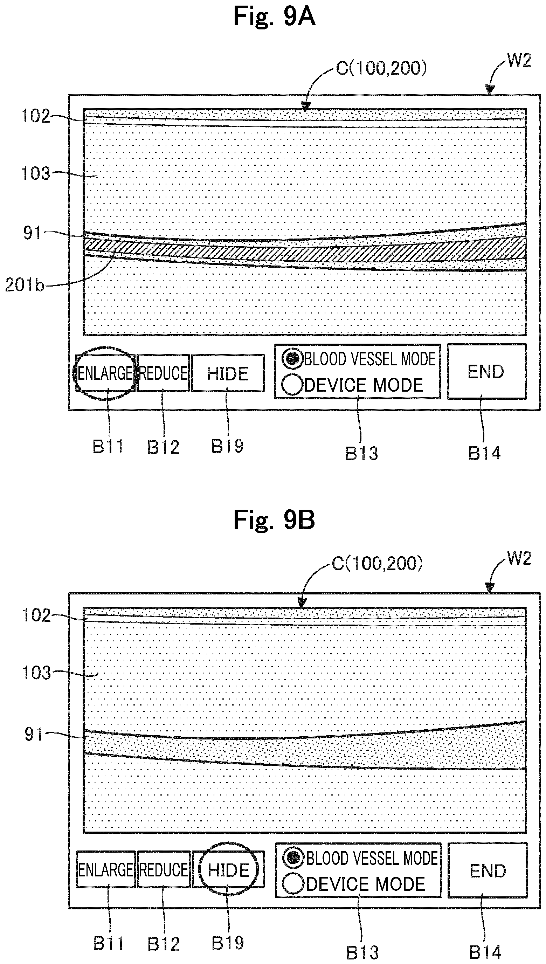

Figs. 9A and 9B are diagrams describing the operation of the guideline 201. Fig. 9A shows an example of the composite image display screen W2 after the enlargement instruction operation, and Fig. 9B shows an example of the composite image display screen W2 after the display switching instruction operation. In step S54 of Fig. 4, the guide image generation portion 53 regenerates the guide image. Specifically, the guide image generation portion 53 performs the following processes b1 to b3 according to the button pressed on the composite image display screen W2 (in other words, the instruction operation acquired in step S52).
(b1) In case the enlargement button B11 is pressed: the guide image generation portion 53 draws a guideline 201b with the width W201 relatively thickened over the entire length according to the enlargement instruction operation caused by pressing the enlargement button B11, and regenerates the guide image 200 in which this guideline 201b is placed at the position of the center line of the blood vessel 91.
(b2) In case the reduction button B12 is pressed: the guide image generation portion 53 draws the guideline 201 with the width W201 relatively narrowed over the entire length according to the reduction instruction operation caused by pressing the reduction button B12, and regenerates the guide image 200 in which this guideline 201 is placed at the position of the center line of the blood vessel 91.
(b3) In case the hide button B19 is pressed: the guide image generation portion 53 changes the transparency of the guideline 201 to a transparency of zero (i.e., the guideline 201 is transparent and cannot be visually recognized) according to the display switching instruction operation by the hide button B19. Note that, when the hide button B19 is pressed, the guide image generation portion 53 may generate the guide image 200 that does not include the guideline 201 instead of setting the transparency of the guideline 201 to zero in the process b3. Further, when the hide button B19 is pressed, the guide image generation portion 53 may skip the step S54 (processing b3) and generate the composite image C including only the ultrasonic echo image 100 in the subsequent step S56.

In step S56 of Fig. 4, the display control portion 54 generates the composite image C in which the ultrasonic echo image 100 generated by the echo image generation portion 52 and the guide image 200 regenerated in step S54 are superimposed, and causes the display portion 60 to display the composite image C. As a result, as shown in Fig. 9A, when the enlargement button B11 is pressed, the guideline 201b with a relatively thick width is displayed. Similarly, when the reduction button B12 is pressed, the guideline 201 with a relatively narrow width is displayed. Further, as shown in Fig. 9B, the guideline 201 is hidden when the hide button B19 is pressed. Note that, in step S54, the guide image generation portion 53 may perform processing of increasing or decreasing the width W201 of the guideline by ±x% (x is a freely selected value) when the enlargement button B11 or the reduction button B12 is pressed once. In this manner, the operator can gradually change the width W201 of the guideline by pressing the enlargement button B11 or the reduction button B12 multiple times until the desired thickness is obtained.

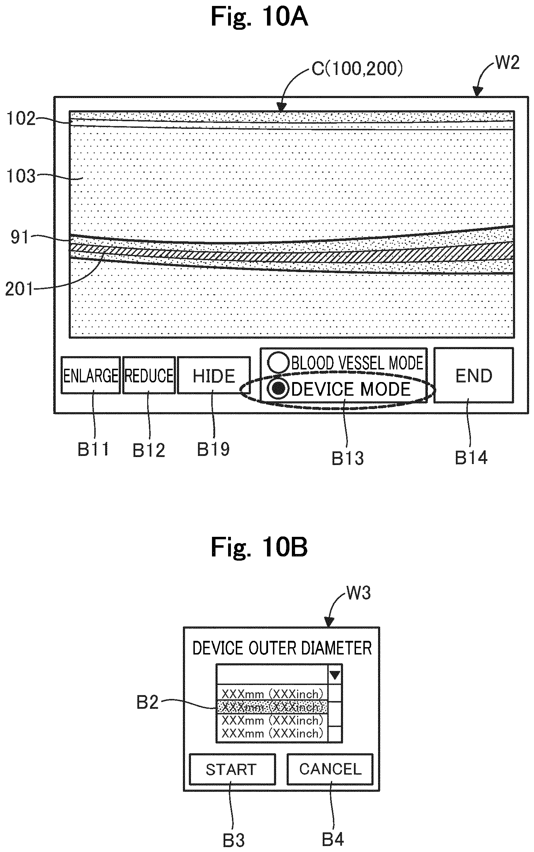

Figs. 10A and 10B are diagrams describing how to change the display mode. Fig. 10A shows an example of the composite image display screen W2, and Fig. 10B shows an example of a selection screen W3. In step S58 of Fig. 4, the display control portion 54 determines whether a display mode changing operation has been performed, that is, whether a mode different from the current mode has been selected in the display mode selection section B13. If the display mode changing operation has not been performed (step S58: NO), the display control portion 54 shifts the processing to step S52 and repeats the above-mentioned processing. If the display mode changing operation has been performed (step S58: YES), the display control portion 54 shifts the processing to step S18. In this case, in step S18, the guide image generation portion 53 acquires the display mode selected in the display mode selection section B13 of the composite image display screen W2.

The processing will be described for the case where the display mode is the device mode. In step S40 of Fig. 4, the guide image generation portion 53 acquires the outer diameter of the medical device (device outer diameter). Specifically, when the shift is made from the guide screen W1 shown in Fig. 5, the guide image generation portion 53 acquires a selection content in the device outer diameter selection section B2 of the guide screen W1 and sets it as the device outer diameter. On the other hand, when the shift is made from the composite image display screen W2 shown in Figs. 10A and 10B, the guide image generation portion 53 displays the selection screen W3 shown in Fig. 10B. Similar to the guide screen W1 in Fig. 5, the selection screen W3 includes the device outer diameter selection section B2, the start button B3, and the cancel button B4. The guide image generation portion 53 acquires the selection content in the device outer diameter selection section B2 of the selection screen W3 and sets it as the device outer diameter.

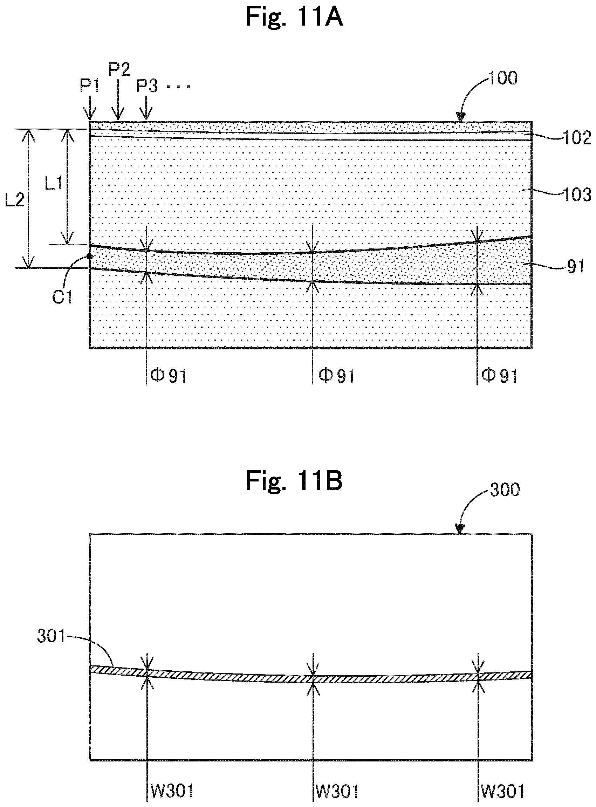

Figs. 11A and 11B are diagrams showing an example of the ultrasonic echo image 100 and a guide image 300. Fig. 11A shows an example of the ultrasonic echo image 100, and Fig. 11B shows an example of the guide image 300. In step S42 of Fig. 4, the guide image generation portion 53 specifies the position of the center line of the blood vessel 91. The details are the same as in step S32.

In step S44 of Fig. 4, the guide image generation portion 53 generates the guide image 300 including a guideline 301 according to the outer diameter of the medical device. Specifically, the guide image generation portion 53 draws the guideline 301 having a constant width W301 according to the outer diameter of the medical device acquired in step S40, and generates an image, as the guide image 300, in which this guideline 301 is placed at the position of the center line of the blood vessel 91 specified in step S42. In this process, the guide image generation portion 53 preferably makes the width W301 of the guideline 301 thicker than the outer diameter of the medical device acquired in step S40. Note that the display format (e.g., hue, saturation, brightness, transparency, etc.) of the guideline 301 can be freely determined. For example, the display format of the guideline 301 in the device mode may be the same as the display format of the guideline 201 in the blood vessel mode, or may be different such that they can be distinguished at a glance. Fig. 11B shows an example of the guide image 300 generated in step S44. In the example of Fig. 11B, the guideline 301 has the constant width W301, unlike the guideline 201 in the blood vessel mode. As described above, the width W301 is changed depending on the outer diameter of the medical device.

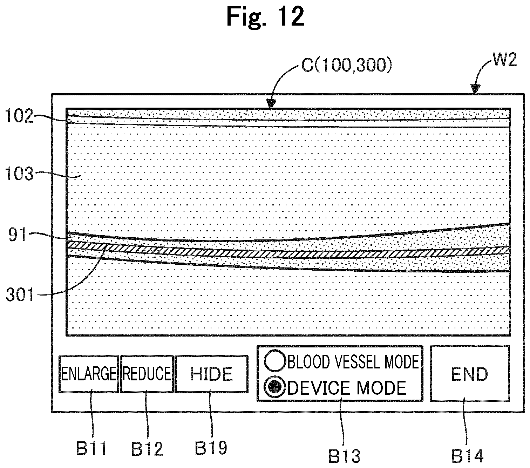

Fig. 12 is a diagram showing an example of the composite image display screen W2. After completing step S44 of Fig. 4, the guide image generation portion 53 shifts the processing to step S50. In the processing contents after step S50, the "guide image 200" may be replaced with the "guide image 300" and the "guideline 201" may be replaced with as the "guideline 301" in the above description. As a result of step S50, as shown in Fig. 12, the display portion 60 displays the composite image display screen W2 including the composite image C in which the ultrasonic echo image 100 and the guide image 300 are superimposed. Note that, also on the composite image display screen W2 shown in Fig. 12, as described above, the operation of the guideline 301 can be performed by pressing the enlargement button B11, the reduction button B12, and the hide button B19, and the switching operation to the blood vessel mode can be performed using the display mode selection section B13.

The guidelines 201 and 301 on the composite image display screen W2 shown in Fig. 8, Figs. 9A and 9B, and Fig. 12 function as a route for delivering the medical device in the blood vessel 91. When the operator inserts a medical device into the blood vessel 91, and the medical device is positioned on the scanning plane SC of the ultrasonic probe 40, the composite image C on the composite image display screen W2 includes the image of the medical device. In this manner, while referring to the composite image C on the composite image display screen W2, the operator delivers the medical device such that the image of the medical device is aligned along the guidelines 201 and 301, so that the operator can effortlessly deliver the medical device along the central axis O of the blood vessel 91.

As described above, according to the medical apparatus 50 of the first embodiment, the display control portion 54 displays the composite image C in which the ultrasonic echo image 100 and the guide images 200 and 300 including the guidelines 201 and 301, respectively, are superimposed, making it possible to guide the medical device through the delivery route in the echo-guided intervention. Further, the guidelines 201 and 301 extend along the center line of the blood vessel 91 appearing in the ultrasonic echo image 100. Thus, simply by delivering the medical device along the guidelines 201 and 301, the operator can deliver the medical device to the target position without the medical device touching the vascular wall. This can improve the safety and efficiency of the procedure. Further, the guidelines 201 and 301 are drawn by the guide image generation portion 53. Thus, the guidelines 201 and 301 are not interrupted on the occurrence of an issue such as the blood vessel 91 appearing in the ultrasonic echo image 100 becoming blurred, or a part of the blood vessel 91 not appearing in the ultrasonic echo image 100, due to, for example, the presence of the tissue with a relatively high reflectance of ultrasonic waves (the bone 92, the calcified lesion, etc.) in the travel direction of the ultrasonic waves emitted from the ultrasonic probe 40. Thus, according to the medical apparatus 50, the safety and efficiency of the procedure can be improved without relying on the information in the ultrasonic echo image (in other words, ultrasonic information).

Further, according to the medical apparatus 50 (blood vessel mode) of the first embodiment, the guide image generation portion 53 changes the width W201 of the guideline 201 according to the inner diameter φ91 of the blood vessel 91. Thus, the width W201 of the guideline 201 can be narrowed in a part where the inner diameter φ91 of the blood vessel 91 is relatively small, and the width W201 of the guideline 201 can be thickened in a part where the inner diameter φ91 of the blood vessel 91 is relatively large (Fig. 8). This allows the operator to quickly recognize, based on the width W201 of the guideline 201, a part where the medical device needs to be advanced carefully (i.e., a part where the inner diameter φ91 of the blood vessel 91 is relatively small) and a part where the medical device can be advanced normally (i.e., a part where the inner diameter φ91 of the blood vessel 91 is relatively large). As a result, according to the medical apparatus 50, the safety and efficiency of the procedure can be further improved.

Further, according to the medical apparatus 50 (blood vessel mode) of the first embodiment, the guide image generation portion 53 sets the width W201 of the guideline 201 within the range of 40% or more and 60% or less of the inner diameter φ91 of the corresponding part of the blood vessel 91. This allows the operator to recognize the guideline 201 at a glance and deliver the medical device along the guideline 201 relatively easily, and makes it possible to reduce the possibility of the medical device touching the inner wall of the blood vessel 91. Further, the width W201 of the guideline 201 is narrow in a part where the inner diameter φ91 of the blood vessel 91 is relatively small, and the width W201 of the guideline 201 is thick in a part where the inner diameter φ91 of the blood vessel 91 is relatively large. This allows the operator to quickly recognize, based on the width W201 of the guideline 201, the part where the medical device needs to be advanced carefully and the part where the medical device may be advanced normally, making it possible to further improve the safety and efficiency of the procedure.

Further, according to the medical apparatus 50 (device mode) of the first embodiment, the guide image generation portion 53 changes the width W301 of the guideline 301 according to the outer diameter of the medical device. Thus, if the outer diameter of the medical device is relatively narrow, the width W301 of the guideline 301 can be made narrow, and if the outer diameter of the medical device is relatively thick, the width W301 of the guideline 301 can be made thick (Fig. 12). This makes it easy for the operator to visually recognize both the medical device and the guideline 301 on the composite image C. As a result, according to the medical apparatus 50, the safety and efficiency of the procedure can be further improved.

Further, according to the medical apparatus 50 (device mode) of the first embodiment, the guide image generation portion 53 makes the width W301 of the guideline 301 thicker than the outer diameter of the medical device. This allows the operator to deliver the medical device to the target position without the medical device touching the vascular wall of the blood vessel 91 simply by delivering the medical device such that the medical device does not protrude from the guideline 301 on the composite image C. This can further improve the safety and efficiency of the procedure.

Further, according to the medical apparatus 50 of the first embodiment, if the ultrasonic information includes the missing part 107 in which the vascular wall of the blood vessel 91 cannot be distinguished from other tissues, the guide image generation portion 53 uses information on the surroundings 108 and 109 of the missing part 107 to interpolate the missing part 107, thereby specifying the position of the center line of the blood vessel 91 (Fig. 7). Thus, even if there is a missing part in the ultrasonic information due to, for example, the presence of tissues with a relatively high reflectance of ultrasonic waves (the bone 92, the calcified lesion, etc.) in the travel direction of the ultrasonic waves emitted from the ultrasonic probe 40, the guide image generation portion 53 can specify the position of the center line of the blood vessel 91 and draw the guidelines 201 and 301.

Further, according to the medical apparatus 50 of the first embodiment, the width of the guidelines 201 and 301 can be changed according to the enlargement instruction operation or the reduction instruction operation by the operator (Figs. 9A and 9B). This can improve the usability of the medical apparatus 50. Further, according to the medical apparatus 50, the blood vessel mode and the device mode can be switched by the switching instruction operation by the operator (Figs. 10A and 10B). This can further improve the usability of the medical apparatus 50.

### <Second embodiment>

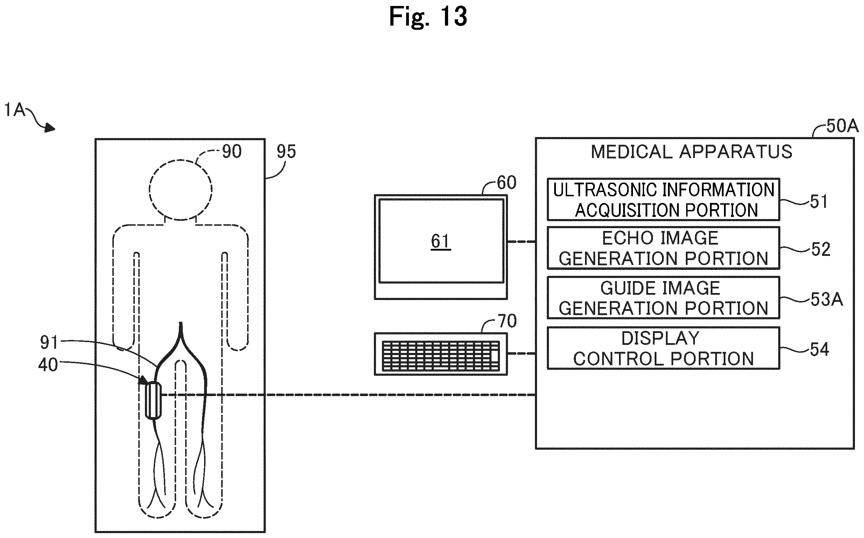

Fig. 13 is an explanatory diagram illustrating a configuration of a medical system 1A according to a second embodiment. In the second embodiment, an example is described in which the display format of a guideline 201A in a guide image 200A is different from that in the first embodiment. The medical system 1A of the second embodiment includes a medical apparatus 50A instead of the medical apparatus 50 in the configuration described in the first embodiment. The medical apparatus 50A includes a guide image generation portion 53A instead of the guide image generation portion 53.

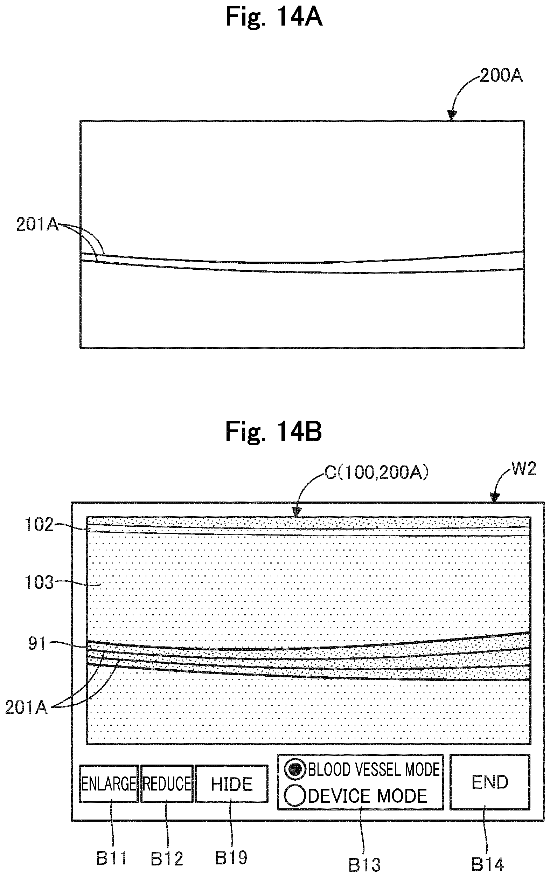

Figs. 14A and 14B are diagrams showing an example of the guide image 200A and the composite image display screen W2. Fig. 14A shows an example of the guide image 200A, and Fig. 14B shows an example of the composite image display screen W2. A processing procedure of the guide processing in the second embodiment is the same as that in Fig. 4. In step S34 of Fig. 4, the guide image generation portion 53A draws the guideline 201A in which an outline is drawn with a line segment of a predetermined color and the inside of the outline is made transparent or translucent, and generates an image, as the guide image 200A, in which this guideline 201A is placed at the position of the center line of the blood vessel 91 specified in the step S32. Note that the guide image generation portion 53A changes the width of the guideline 201A according to the change in the inner diameter φ91 of the blood vessel 91, similarly to the first embodiment.

As a result, the guide image 200A shown in Fig. 14A is generated. The guide image 200A includes the guideline 201A in which only the outline is drawn as a solid line and the inside of the outline is made transparent (or translucent). Fig. 14B shows the composite image display screen W2 including the composite image C generated using the guide image 200A. When the operator inserts a medical device into the blood vessel 91, and the medical device is positioned on the scanning plane SC of the ultrasonic probe 40, the composite image C on the composite image display screen W2 also includes the image of the medical device. In the present embodiment, the inside of the guideline 201A is transparent or translucent. Thus, even if the guideline 201A overlaps with the image of the medical device, the operator can easily recognize the image of the medical device.

Note that, similarly, in step S44 of Fig. 4, the guide image generation portion 53A draws the guideline 301 in which an outline is drawn with a line segment of a predetermined color and the inside of the outline is made transparent or translucent, and generates an image, as the guide image 300, in which this guideline 301 is placed at the position of the center line of the blood vessel 91 specified in the step S32. Note that the guide image generation portion 53A determines the width of the guideline 301 according to the outer diameter of the medical device, similarly to the first embodiment.

As described above, the display format of the guidelines 201A and 301 can be modified in various manners. The inside of the outline may be made transparent or translucent, or may be subjected to known image processing to improve the visibility of the medical device. Further, on the composite image display screen W2, the guidelines 201 and 301 in the display format described in the first embodiment and the guidelines 201A and 301 in the display format described in the second embodiment may be made switchable according to the operator's preference. Such a medical apparatus 50A of the second embodiment can also achieve the same effects as the first embodiment described above. Further, according to the medical apparatus 50A of the second embodiment, the guide image generation portion 53A draws the guidelines 201A and 301 in which the outline is drawn with a line segment of a predetermined color and the inside of the outline is made transparent or translucent. This can prevent the guidelines 201A and 301 from interfering with the recognition of the medical device on the composite image C. As a result, the efficiency of the procedure can be further improved, and the usability of the medical apparatus 50A can be further improved.

### <Third embodiment>

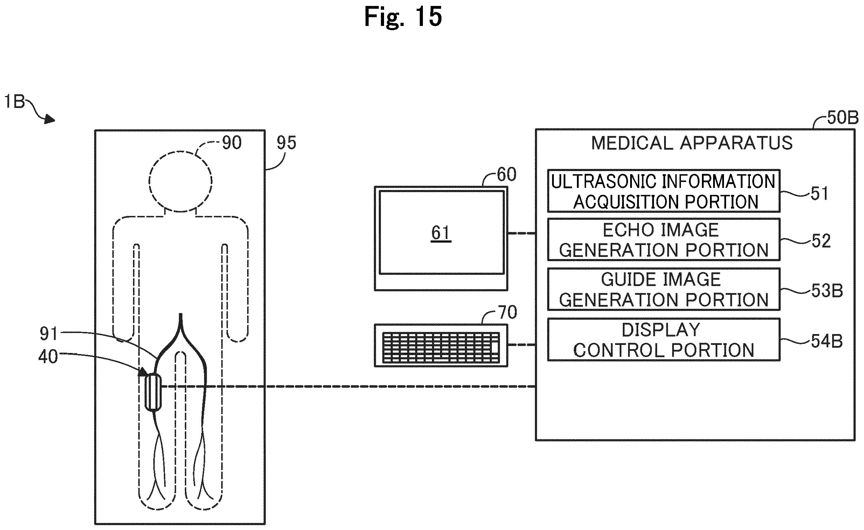

Fig. 15 is an explanatory diagram illustrating a configuration of a medical system 1B according to a third embodiment. In the third embodiment, an example is described in which the medical apparatus 50B automatically specifies and acquires the outer diameter of the medical device. The medical system 1B of the third embodiment includes a medical apparatus 50B instead of the medical apparatus 50 in the configuration described in the first embodiment. The medical apparatus 50B includes a guide image generation portion 53B instead of the guide image generation portion 53 and a display control portion 54B instead of the display control portion 54.

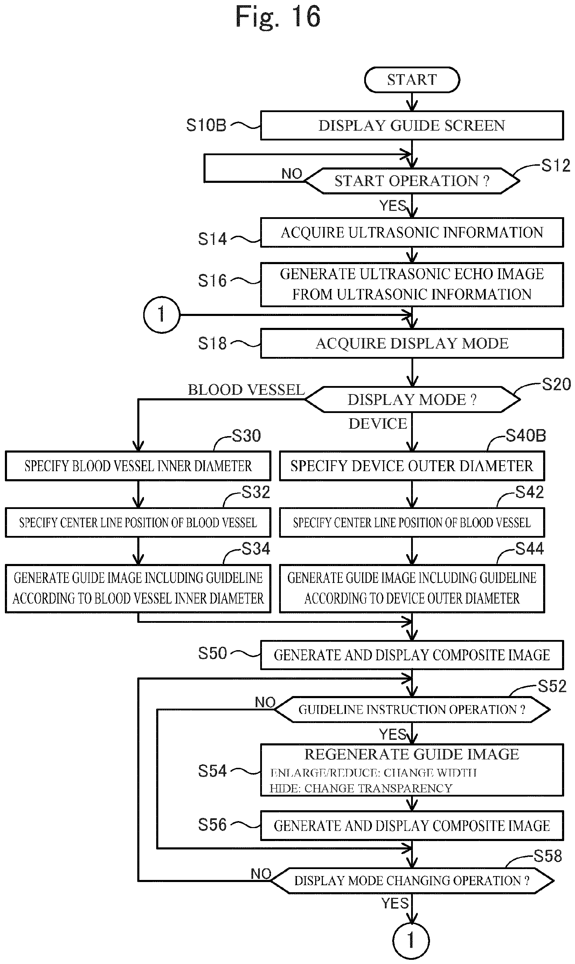

Fig. 16 is a flowchart showing an example of a processing procedure of the guide processing of the third embodiment. The guide processing shown in Fig. 16 is the same as the first embodiment described in Fig. 4, except that a step S10B is executed instead of the step S10, and a step S40B is executed instead of the step S40. In step S10B of Fig. 16, the display control portion 54B causes the display portion 60 to display the guide screen W1. In this step, the display control portion 54B generates and displays the guide screen W1 that does not include the device outer diameter selection section B2 in the guide screen W1 described in Fig. 5.

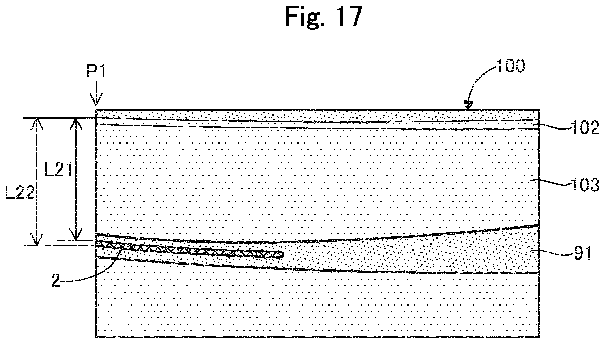

Fig. 17 is a diagram describing specification of the device outer diameter. In step S40B of Fig. 16, the guide image generation portion 53B uses the ultrasonic information acquired by the ultrasonic information acquisition portion 51 to specify the outer diameter of a medical device 2 (Fig. 17). Specifically, the guide image generation portion 53B obtains distances L21 and L22 at a given point P1.
(c1) Distance L21: distance from the surface of the skin 102 to the proximal surface of the medical device 2.
(c2) Distance L22: distance from the surface of the skin 102 to the distal surface of the medical device 2.

Then, the guide image generation portion 53B specifies and acquires a value obtained by subtracting the distance L21 from the distance L22 as the outer diameter of the medical device 2. In the illustrated example, the outer diameter of the medical device 2 is measured only at one point, the point P1. However, the guide image generation portion 53B may measure the outer diameter of the medical device 2 by performing the same processing at multiple different points and specify the outer diameter of the medical device 2 using their statistical values (e.g., an average value, a mode, etc.).

Other steps in Fig. 16 are the same as in Fig. 4. As described above, the guide processing procedure can be modified in various manners. For example, the guide image generation portion 53B may automatically specify the outer diameter of the medical device 2 without relying on input from the operator. In such a medical apparatus 50B of the third embodiment, the same effects as those of the first embodiment described above can be achieved. Further, according to the medical apparatus 50B of the third embodiment, the outer diameter of the medical device 2 can be automatically acquired. Thus, human errors due to selection errors can be prevented, and the usability of the medical apparatus 50B can be further improved.

### <Modifications of present embodiments>

In the above embodiments, a part of the configurations achieved by hardware may be replaced with software, or conversely, a part of the configurations achieved by software may be replaced by hardware. Further, the present invention is not limited to the above-mentioned embodiments and can be implemented in various aspects without departing from the scope of the invention. For example, the following modifications can be made.

### [First modification]

In the first to third embodiments described above, the configurations of the medical systems 1, 1A, and 1B are illustrated. However, the configurations of the medical systems 1, 1A, and 1B can be modified in various manners. For example, in the medical system 1, at least some of the ultrasonic probe 40, the medical apparatuses 50, 50A, and 50B, the display portion 60, and the operation portion 70 may be configured as an integrated device. For example, the medical system 1 may include other apparatuses such as a magnetic sensor array, a CT apparatus, an MRI apparatus, an electrocardiograph, and an X-ray imaging apparatus.

### [Second modification]

In the first to third embodiments described above, examples of the configurations of the medical apparatuses 50, 50A, and 50B, and examples of the guide processing executed by the medical apparatuses 50, 50A, and 50B are illustrated. However, these can be modified in various manners. For example, the processing content in the guide processing can be modified in various manners. The execution order of each step may be changed, a part of the processing in each step may be omitted, or other steps not described may be added.

For example, in step S10, the display control portion 54 may perform the guidance and acquire the operation from the operator by a means other than the guide screen W1. Examples of other means include a combination of voice guidance and a foot switch. For example, in step S34, the guide image generation portion 53 may set the width W201 of the guideline 201 to be less than 40% or greater than 60% of the inner diameter φ91 of the corresponding part of the blood vessel 91. For example, the guide image generation portion 53 may set the width W201 of the guideline 201 within a range of 30% or more and 70% or less of the inner diameter φ91 of the corresponding part of the blood vessel 91.

For example, only one of the processing (steps S30 to S34) in which the display mode is the blood vessel mode and the processing (steps S40 to S44) in which the display mode is the device mode may be executed, and the other processing may not be executed. For example, the processing related to the guideline operation (steps S52 to S56) may be omitted. In this case, the enlargement button B11, the reduction button B12, and the hide button B19 on the composite image display screen W2 can be omitted. For example, the processing related to the display mode change (step S58) may not be executed. In this case, the display mode selection section B13 on the composite image display screen W2 can be omitted.

### [Third modification]

The configurations of the medical systems 1, 1A, and 1B of the first to third embodiments described above and the configurations of the first and second modifications described above may be combined as appropriate. For example, in the configuration that automatically acquires the device outer diameter described in the third embodiment, the display format of the guidelines 201A and 301 described in the second embodiment may be adopted.

In the above, the present aspects are described based on the embodiments and modifications. However, the embodiments of the above-mentioned aspects are provided merely for clear understanding of the present aspects, and should not be construed as limiting to the present aspects. The present aspects can be altered or modified without departing from the spirit thereof and the claims. The present aspects include any equivalents thereto. Further, the technical features thereof, if not indicated as essential in the present specification, may be appropriately deleted.

### DESCRIPTION OF REFERENCE NUMERALS

1, 1A, 1B Medical systems
2 Medical device
40 Ultrasonic probe
41 Main body portion
42 Handle portion
44 Ultrasonic sensor
50, 50A, 50B Medical apparatuses
51 Ultrasonic information acquisition portion
52 Echo image generation portion
53, 53A, 53B Guide image generation portions
54, 54B Display control portions
60 Display portion
61 Display screen
70 Operation portion
90 Human body
91 Blood vessel
92 Bone
93 Muscle
94 Fat
95 Bed
100 Ultrasonic echo image
102 Skin
103 Subcutaneous tissue
107 Missing part
108 Surrounding
200, 200A Guide images
201, 201A, 201b Guidelines
300 Guide image
301 Guideline
441 Element group
442 Element group
443 Element group
C Composite image
SC Scanning plane
W1 Guide screen
W2 Composite image display screen

## Claims

1. A medical apparatus comprising:
an ultrasonic information acquisition portion that acquires ultrasonic information inside a living body, the ultrasonic information being obtained from an ultrasonic probe that includes an ultrasonic sensor and applies ultrasonic waves into the living body;
an echo image generation portion that generates an ultrasonic echo image representing a longitudinal section of a blood vessel from the ultrasonic information;
a guide image generation portion that generates a guide image including a guideline extending along a center line of the blood vessel appearing in the ultrasonic echo image from the ultrasonic information; and
a display control portion that generates and displays a composite image in which the ultrasonic echo image and the guide image are superimposed.

2. The medical apparatus according to claim 1, wherein the guide image generation portion changes a width of the guideline according to an inner diameter of the blood vessel.

3. The medical apparatus according to claim 2, wherein the guide image generation portion sets the width of the guideline within a range of 40% or more and 60% or less of the inner diameter of a corresponding part of the blood vessel.

4. The medical apparatus according to any one of claims 1 to 3, wherein the guide image generation portion changes the width of the guideline according to an outer diameter of a medical device to be inserted into the blood vessel.

5. The medical apparatus according to claim 4, wherein the guide image generation portion makes the width of the guideline thicker than the outer diameter of the medical device.

6. The medical apparatus according to any one of claims 1 to 5, further comprising an operation acquisition portion that acquires an enlargement instruction operation or a reduction instruction operation of the width of the guideline from a user of the medical apparatus, wherein:
the guide image generation portion regenerates the guide image in which the width of the guideline is thickened in response to the enlargement instruction operation, and regenerates the guide image in which the width of the guideline is narrowed in response to the reduction instruction operation; and
the display control portion generates and displays the composite image in which the ultrasonic echo image and the regenerated guide image are superimposed.

7. The medical apparatus according to any one of claims 1 to 6, wherein the guide image generation portion generates the guide image including the guideline in which an outline is drawn as a line segment of a predetermined color and an inside of the outline is made transparent or translucent.
